# EUROPÄISCHE PATENTSCHRIFT

(11) **EP 3 023 066 B1**
(45) Veröffentlichungstag und Bekanntmachung des Hinweises auf die Patenterteilung: **26.02.2020**
(21) Anmeldenummer: 15195766.9
(22) Anmeldetag: 23.11.2015
(51) Int. Cl.: A61B 17/70

(54) **PEDIKELSCHRAUBENSYSTEM UND WIRBELSÄULENSTABILISIERUNGSSYSTEM**
PEDICLE SCREW SYSTEM AND SPINAL COLUMN-STABILIZING SYSTEM
SYSTÈME DE VIS PÉDICULAIRE ET SYSTÈME DE STABILISATION DE COLONNE VERTÉBRALE

(30) Priorität: 24.11.2014 DE 102014117175
(43) Veröffentlichungstag der Anmeldung: 25.05.2016
(73) Patentinhaber: AESCULAP AG, 78532 Tuttlingen (DE)
(72) Erfinder: Beger, Jens, 78532 Tuttlingen (DE); Stoerk, Claudia, 78576 Emmingen (DE); Gassner, Stefan, 78194 Immendingen-Hattingen (DE); Krüger, Sven, 78647 Trossingen (DE)
(74) Vertreter: Hoeger, Stellrecht & Partner Patentanwälte mbB

(56) Entgegenhaltungen:
- DE-A1-102006 055 599
- FR-A1- 2 761 876
- US-A1- 2008 086 131
- US-A1- 2010 305 616
- US-A1- 2013 030 474

## Beschreibung

Die vorliegende Erfindung betrifft ein Pedikelschraubensystem umfassend eine Pedikelschraube mit einem Schraubenschaft, welcher ein Außengewinde aufweist, und einem kugelgelenkig am Schraubenschaft gelagerten Schraubenkopf, welcher Schraubenkopf eine Verbindungselementaufnahme für ein Verbindungselement eines Wirbelsäulenstabilisierungssystems umfasst, das Pedikelschraubensystem ferner umfassend eine Knochenausrichteinrichtung und eine Kopplungseinrichtung umfasst zum kraft- und/oder formschlüssigen Koppeln der Knochenausrichteinrichtung und des Schraubenschafts in einer Ausrichtstellung.

Die Erfindung betrifft ferner ein Wirbelsäulenstabilisierungssystem umfassend mindestens zwei Knochenschrauben und mindestens ein an den mindestens zwei Knochenschrauben festlegbares Verbindungselement.

Pedikelschrauben und Wirbelsäulenstabilisierungssysteme der eingangs beschriebenen Art sind beispielsweise aus der DE 10 2013 100 574 A1 bekannt. Mit ihnen lassen sich beispielsweise bei Deformitätenoperationen deformierte Wirbelsäulen durch entsprechende Implantation und Ausrichtung von Pedikelschrauben in eine gewünschte Form bringen und in dieser fixieren. Zur Ausrichtung einzelner, fehlgestellter Wirbel werden die Kräfte für die Korrekturmanöver über die Pedikelschrauben in den jeweiligen Wirbel eingeleitet.

Bei Pedikelschraubensystemen, bei denen ein Verbindungselement von oben in eine entsprechende Verbindungselementaufnahme am Schraubenkopf eingesetzt werden kann, also bei sogenannten "Tulpen"-Systemen, ist eine Krafteinleitung nicht möglich, wenn die Pedikelschraube in Form einer Polyaxialschraube ausgebildet ist. Eine Krafteinleitung ist nur möglich, wenn der Schraubenkopf relativ zum Schraubenschaft unbeweglich oder maximal um eine Achse verschwenkbar ist, es sich also bei der Pedikelschraube um eine sogenannte Monoaxialschraube handelt. Der Schraubenkopf wird dabei in einer Ebene bewegt, welche senkrecht zur Achse, um die verschwenkt wird, verläuft, so dass die Monoaxialschraube in diesem Sinne auch als Uniplanarschraube bezeichnet werden kann. Bei Polyaxialschrauben hingegen, die das Einsetzen des Verbindungselements, beispielsweise eines Stabs, deutlich dadurch vereinfachen, dass der Schraubenkopf beliebig relativ zum Schraubenschaft orientiert werden kann, ist eine solche Krafteinleitung und Korrektur einer Ausrichtung eines Wirbels nicht oder nur rudimentär möglich. Insbesondere ist es nicht möglich, die Technik der segmentalen Derotation bei Polyaxialschrauben anzuwenden. Diese ist nur bei direkter Krafteinleitung in die Pedikelschraube, wie es insbesondere die beschriebenen Monoaxialschrauben ermöglichen, realisierbar.

Die US 2013/0030474 A1 offenbart Knochenschrauben und Knochenschraubensysteme. Aus der US 2010/0305616 A1 ist eine Befestigungsvorrichtung für die Wirbelsäule bekannt. Befestigungen mit Knochenschrauben sind in der US 2008/0086131 A1 beschrieben. Ein lumbales Osteosyntheseinstrument ist in der FR 2 761 876 A1 offenbart. Orthopädische Befestigungsvorrichtungen und Vorrichtungen zur Korrektur einer Wirbelsäulendeformität sind aus der DE 10 2006 055 599 A1 bekannt.

Es ist daher eine Aufgabe der vorliegenden Erfindung, ein Pedikelschraubensystem und ein Wirbelsäulenstabilisierungssystem der eingangs beschriebenen Art so weiter zu bilden, dass deren Handhabung verbessert wird.

Diese Aufgabe wird bei einem Pedikelschraubensystem der eingangs beschriebenen Art erfindungsgemäß dadurch gelöst, dass der Kopplungsvorsprung in der Ausrichtstellung formschlüssig oder im Wesentlichen formschlüssig in die Kopplungsaufnahme eingreift, dass der Kopplungsvorsprung in Form eines Ringflansches ausgebildet ist, dass die Kopplungsaufnahme in Form einer Ausnehmung der Knochenausrichteinrichtung ausgebildet ist, dass die Knochenausrichteinrichtung mindestens eine Kupplungseinrichtung zum temporären kraft- und/oder formschlüssigen Kuppeln mit einem Ausrichtinstrument in einer Kuppelstellung aufweist, dass die Kupplungseinrichtung mindestens ein Kupplungselement zum kraft- und/oder formschlüssigen Kuppeln mit dem Ausrichtinstrument in einer Kuppelstellung umfasst, dass das mindestens eine Kupplungselement in Form eines Kupplungsvorsprungs und/oder in Form einer Kupplungsausnehmung ausgebildet ist, dass das mindestens eine Kupplungselement ein Gewinde umfasst, insbesondere ein Außen- oder ein Innengewinde, oder in Form eines Rastelements ausgebildet ist, dass das Pedikelschraubensystem ein Ausrichtinstrument zum temporären kraft- und/oder formschlüssigen Kuppeln mit der Kupplungseinrichtung umfasst und dass das Ausrichtinstrument distalseitig ein Kupplungsende aufweist zum kraft- und/oder formschlüssigen Kuppeln mit der Kupplungseinrichtung.

Die erfindungsgemäß vorgeschlagene Weiterbildung ermöglicht es dem Operateur insbesondere, über die Knochenausrichteinrichtung Kräfte zum Bewegen, insbesondere Rotieren, deformierter Wirbel einer Wirbelsäule direkt auf den Schraubenschaft einzuleiten, wenn die Kopplungseinrichtung des Pedikelschraubensystems die Ausrichtstellung einnimmt. Das vorgeschlagene Pedikelschraubensystem vereinigt somit einerseits die Vorteile von Polyaxialschrauben, die eine beliebige Ausrichtung des Schraubenkopfs relativ zum Schraubenschaft ermöglichen, um das Einsetzen des Verbindungselements in die Verbindungselementaufnahme am Schraubenkopf zu erleichtern, und andererseits die Vorteile von starren Schrauben oder Monoaxialschrauben, die das Einleiten von Kräften auf den Schraubenschaft zur Ausrichtung eines Wirbelkörpers, in den die Pedikelschraube eingeschraubt ist, ermöglichen. Durch die Kopplungseinrichtung können insbesondere eine axiale und/oder drehfeste Verbindung zwischen der Knochenausrichteinrichtung und dem Schraubenschaft hergestellt und somit über die Knochenausrichteinrichtung eine indirekte Kraftübertragung beispielsweise von einem Ausrichtinstrument auf den Schraubenschaft ermöglicht werden. Besonders einfach und kostengünstig ausbilden lässt sich das Pedikelschraubensystem dadurch, dass die Knochenausrichteinrichtung in Form einer Knochenplatte ausgebildet ist. Insbesondere kann die Knochenplatte eine Knochenanlagefläche aufweisen, die eine möglichst flächige Anlage an einen Knochen ermöglicht. Insbesondere kann sie an entsprechend einer anatomischen Krümmung des Knochens, an den sie angelegt werden soll, geformt oder an diese angepasst sein. Beispielsweise kann eine patientenindividuelle Krümmung der Knochenplatte auf Basis von Patientendaten, insbesondere CT-Daten oder andere, durch bildgebende Verfahren ermittelten Daten, hergestellt werden. Zur Ausbildung der insbesondere patientenindividuellen Knochenplatte können zum Beispiel spanende, insbesondere Fräsen, oder generative Herstellungsverfahren, insbesondere Lasersintern, zur Anwendung kommen. Gemäß der Erfindung ist vorgesehen, dass die Kopplungseinrichtung erste und zweite Kopplungselemente umfasst, welche in der Ausrichtstellung kraft- und/oder formschlüssig in Eingriff stehen und einerseits an der Knochenausrichteinrichtung und andererseits am Schraubenschaft angeordnet oder ausgebildet sind. Mit einer derart ausgebildeten Kopplungseinrichtung ist es auf einfache Weise möglich, eine axiale und/oder drehfeste Verbindung zwischen der Knochenausrichteinrichtung und dem Schraubenschaft herzustellen, insbesondere in der Ausrichtstellung. Bevor das Pedikelschraubensystem die Ausrichtstellung einnimmt, können die ersten und zweiten Kopplungselemente in Abhängigkeit von ihrer konkreten Ausgestaltung beispielsweise gegeneinander rotiert werden, insbesondere polyaxial durch Ausbildung eines Kugelgelenks zwischen dem ersten und zweiten Kopplungselement, um dann in ihrer Bewegungsfreiheit relativ zueinander ganz oder teilweise eingeschränkt zu werden. Beispielsweise können die ein Kugelgelenk ausbildenden ersten und zweiten Kopplungselemente bei vollständig oder nahezu vollständig in den Knochen eingeschraubter Pedikelschraube gegeneinander gepresst oder aktiv durch ein zusätzliches Blockierelement relativ zueinander blockiert werden. Vorteilhaft ist es, dass das erste Kopplungselement in Form eines Kopplungsvorsprungs und dass das zweite Kopplungselement in Form einer zum Kopplungsvorsprung korrespondierenden Kopplungsaufnahme ausgebildet ist. Beispielsweise kann der Kopplungsvorsprung an der Knochenausrichteinrichtung oder am Schraubenschaft ausgebildet sein. Entsprechend kann korrespondierend die Kopplungsaufnahme am Schraubenschaft oder an der Knochenausrichteinrichtung ausgebildet sein. Eine axiale und/oder eine drehfeste Verbindung zwischen der Knochenausrichteinrichtung und dem Schraubenschaft lässt sich auf einfache Weise dadurch realisieren, dass der Kopplungsvorsprung in der Ausrichtstellung formschlüssig oder im Wesentlichen formschlüssig in die Kopplungsaufnahme eingreift. Auf besonders einfache und kostengünstige Weise herstellen lässt sich das Pedikelschraubensystem dadurch, dass der Kopplungsvorsprung in Form eines Ringflansches ausgebildet ist. Beispielsweise kann dieser einstückig mit dem Schraubenschaft ausgebildet sein. Vorteilhaft ist es, dass die Kopplungsaufnahme in Form einer Ausnehmung der Knochenausrichteinrichtung ausgebildet ist. So kann der Kopplungsvorsprung des Schraubenschafts auf einfache Weise in die Kopplungsaufnahme eingreifen. Um beispielsweise mit einem Ausrichtrinstrument einen Wirbel mit daran festgeschraubter Pedikelschraube auf einfache Weise ausrichten zu können, ist es günstig, wenn die Knochenausrichteinrichtung mindestens eine Kupplungseinrichtung zum temporären kraft- und/oder formschlüssigen Kuppeln mit einem Ausrichtinstrument in einer Kuppelstellung aufweist. Die Kupplungseinrichtung ermöglicht es also einem Operateur, bei Bedarf ein Ausrichtinstrument mit der Knochenausrichteinrichtung in Eingriff zu bringen und so mit dem Ausrichtinstrument eine Kraft zum Ausrichten des Wirbelkörpers über die Knochenausrichteinrichtung auf den Schraubenschaft und somit auf den Wirbel zu übertragen. Um ein einfaches Kuppeln mit unterschiedlichsten Ausrichtinstrumenten zu ermöglichen, ist es günstig, dass das mindestens eine Kupplungselement in Form eines Kupplungsvorsprungs und/ oder in Form einer Kupplungsausnehmung ausgebildet ist. Dies bedeutet insbesondere auch, dass das mindestens eine Kupplungselement teilweise als Kupplungsvorsprung und teilweise als Kupplungsausnehmung ausgebildet sein kann. Beispielsweise kann ein Ausrichtinstrument mit einem freien Ende in eine Kupplungsausnehmung eingreifen oder mit einer Ausnehmung am freien Ende einen Kupplungsvorsprung an der Knochenausrichteinrichtung aufnehmen, um vorzugsweise eine axiale und/oder drehfeste Kupplung zwischen dem Ausrichtinstrument und der Knochenausrichteinrichtung zu erreichen. Um eine definierte Verbindung zwischen der Knochenausrichteinrichtung und einem Ausrichtinstrument zu ermöglichen, ist es vorteilhaft, dass das mindestens eine Kupplungselement ein Gewinde umfasst oder in Form eines Rastelements ausgebildet ist. Beispielsweise kann das Gewinde in Form eines Außen- oder in Form eines Innengewindes ausgebildet sein. Das Ausrichtinstrument kann so auf einfache Weise mit der Knochenausrichteinrichtung verschraubt werden.

Eine einfache Form der Verbindung wird erreicht, wenn das Kupplungselement in Form Rastelements ausgebildet ist, sodass auf einfache Weise ein Verrasten zwischen dem Ausrichtinstrument und der Knochenausrichteinrichtung möglich ist, um diese axial und/oder drehfest miteinander zu kuppeln. Vorteilhafterweise umfasst das Pedikelschraubensystem ein Ausrichtinstrument zum temporären kraft- und/oder formschlüssigen Kuppeln mit der Kupplungseinrichtung. Mit dem Ausrichtinstrument kann in der beschriebenen Weise eine Kraft zum Ausrichten eines Wirbels über die Knochenausrichteinrichtung auf den Schraubenschaft ausgeübt werden. Vorzugsweise weist das Ausrichtinstrument distalseitig ein Kupplungsende auf zum kraft- und/oder formschlüssigen Kuppeln mit der Kupplungseinrichtung. Ein derart ausgebildetes Kupplungsende ermöglicht es insbesondere auf einfache und sicherere Weise, das Ausrichtinstrument beispielsweise axial und/oder drehfest mit der Knochenausrichteinrichtung zu kuppeln.

Um eine Rotation der Knochenausrichteinrichtung am Knochen, an dem sie anliegt, zu verhindern oder die Gefahr hierfür zu reduzieren, ist es vorteilhaft, wenn die Knochenausrichteinrichtung mindestens ein Knochenverankerungselement trägt oder umfasst.

Günstigerweise ist das mindestens eine Knochenverankerungselement in Form eines Knochenpins oder eines Knochenzahns ausgebildet. Diese können insbesondere von der in Form einer Knochenplatte ausgebildeten Knochenausrichteinrichtung abstehend ausgebildet sein.

Um eine besonders stabile Knochenausrichteinrichtung zu erhalten, ist es günstig, wenn das mindestens eine Knochenverankerungselement einstückig mit der Knochenausrichteinrichtung ausgebildet ist.

Vorzugsweise ist der Kopplungsvorsprung einstückig mit dem Schraubenschaft ausgebildet oder kraft- und/oder stoffschlüssig mit dem Schraubenschaft verbunden. Beispielsweise kann der Kopplungsvorsprung mit dem Schraubenschaft verpresst, verklebt, verlötet oder verschweißt sein. Insbesondere ermöglicht es der Kopplungsvorsprung, Kräfte von der Knochenausrichteinrichtung über den Kopplungsvorsprung auf den Schraubenschaft einzuleiten.

Für die Ausbildung einer Polyaxialschraube ist es günstig, wenn der Schraubenschaft einen Gelenkkopf aufweist und wenn der Schraubenkopf eine zum Gelenkkopf korrespondierende Gelenkkopfaufnahme zur Ausbildung eines Kugelgelenks im Zusammenwirken mit dem Gelenkkopf aufweist. Diese Ausgestaltung ermöglicht es insbesondere, den Schraubenkopf um einen Mittelpunkt eines beispielsweise kugelförmigen Gelenkkopfs relativ zum Schraubenschaft zu rotieren und auszurichten.

Um einen möglichst kompakten Aufbau der Pedikelschraube zu ermöglichen, ist es vorteilhaft, wenn der Kopplungsvorsprung zwischen dem Außengewinde und dem Gelenkkopf angeordnet ist.

Vorzugsweise ist ein Außendurchmesser des Kopplungsvorsprungs größer ist als ein maximaler Außendurchmesser des Außengewindes. Dies ermöglicht es insbesondere, den Kopplungsvorsprung auch als Anschlagelement zu nutzen, um eine definierte Kopplung zwischen dem Schraubenschaft und der Knochenausrichteinrichtung zu erreichen.

Günstigerweise grenzt der Kopplungsvorsprung direkt an das Außengewinde an. Auf diese Weise kann insbesondere erreicht werden, dass der Schraubenschaft auch ohne Verwendung der Knochenausrichteinrichtung bis zum Kopplungsvorsprung in einen Knochen eingeschraubt werden kann.

Ferner kann vorgesehen sein, dass die Knochenausrichteinrichtung eine Schraubenschaftaufnahme aufweist, in die der Schraubenschaft in der Ausrichtstellung mindestens teilweise eingreift. Beispielsweise kann der Schraubenschaft die Schraubenschaftaufnahme in der Ausrichtstellung auch durchsetzen. So kann eine zusätzliche Optimierung der Kopplung zwischen der Knochenausrichteinrichtung und dem Schraubenschaft erreicht werden.

Um den Schraubenschaft in die Schraubenschaftaufnahme auf einfache Weise einführen zu können, ist es günstig, wenn die Schraubenschaftaufnahme in Form einer Durchbrechung der Knochenausrichteinrichtung ausgebildet ist. Beispielsweise kann die Durchbrechung in Form einer Bohrung ausgebildet sein. Alternativ ist es auch möglich, die Schraubenschaftaufnahme mit einem Querschnitt auszubilden, welcher eine N-zählige Symmetrie aufweist. So kann insbesondere auf einfache Weise eine drehfeste Kopplung zwischen der Knochenausrichteinrichtung und dem Schraubenschaft hergestellt werden.

Für einen möglichst kompakten Aufbau der Pedikelschraube ist es günstig, wenn die Kopplungsaufnahme direkt an die Schraubenschaftaufnahme angrenzt. Durch entsprechende Formgebung der Kopplungsaufnahme und der Schraubenschaftaufnahme können so auf einfache Weise sowohl eine axiale Fixierung als auch eine drehfeste Verbindung in Umfangsrichtung hergestellt werden.

Vorzugsweise weist die Kopplungsaufnahme einen größeren Innendurchmesser auf als die Schraubenschaftaufnahme. Auf diese Weise kann die Schraubenschaftaufnahme einen Anschlag für den Kopplungsvorsprung bilden, wenn dieser in die Kopplungsaufnahme eingreift.

Gemäß einer weiteren bevorzugten Ausführungsform der Erfindung kann vorgesehen sein, dass die Kopplungseinrichtung eine Längsachse definiert, welche parallel zu einer Schraubenschaftlängsachse des Schraubenschafts verläuft oder mit dieser einen Neigungswinkel einschließt. Beispielsweise kann die Knochenausrichteinrichtung in Form einer Knochenplatte ausgebildet sein, die eine Ebene definiert. Bezogen auf diese kann dann eine Kopplung zwischen Knochenausrichteinrichtung und dem Schraubenschaft erreicht werden, bei der die Schraubenschaftlängsachse senkrecht zu der von der Knochenplatte definierten Ebene ausgerichtet oder relativ zu dieser geneigt ist. Abhängig von der Form eines Wirbels können beispielsweise unterschiedlich ausgebildete Knochenausrichteinrichtungen in Form eines Satzes von Knochenausrichteinrichtungen bereitgestellt werden, um einem Operateur eine optimale Auswahl zu bieten, um die für den Patienten bestmögliche Knochenausrichteinrichtung zu wählen, damit er die deformierte Wirbelsäule wieder in eine gewünschte Form bringen kann.

Um ein einfaches Kuppeln mit unterschiedlichsten Ausrichtinstrumenten zu ermöglichen, ist es günstig, wenn das mindestens eine Kupplungselement in Form eines Kupplungsvorsprungs und/oder in Form einer Kupplungsausnehmung ausgebildet ist. Dies bedeutet insbesondere auch, dass das mindestens eine Kupplungselement teilweise als Kupplungsvorsprung und teilweise als Kupplungsausnehmung ausgebildet sein kann. Beispielsweise kann ein Ausrichtinstrument mit einem freien Ende in eine Kupplungsausnehmung eingreifen oder mit einer Ausnehmung am freien Ende einen Kupplungsvorsprung an der Knochenausrichteinrichtung aufnehmen, um vorzugsweise eine axiale und/oder drehfeste Kupplung zwischen dem Ausrichtinstrument und der Knochenausrichteinrichtung zu erreichen.

Auf besonders einfache Weise herstellen lässt sich die Kupplungseinrichtung, wenn die Kupplungsausnehmung in Form eines Sacklochs oder einer Durchbrechung ausgebildet ist. Insbesondere kann die Durchbrechung in Form einer Bohrung ausgebildet sein.

Gemäß einer besonders bevorzugten Ausführungsform der Erfindung kann vorgesehen sein, dass das Kupplungsende in Form eines Rastelements ausgebildet ist oder ein Gewinde umfasst, welches korrespondierend zum Gewinde des mindestens einen Kupplungselements ausgebildet ist. Das Kupplungsende in Form Rastelements kann auf einfache Weise mit einem korrespondierend ausgebildeten Rastelement an der Knochenausrichteinrichtung rastend in Eingriff gebracht werden, um eine axiale und/oder drehfeste Verbindung schnell und einfach herzustellen. Alternativ kann das ein Gewinde aufweisendes Kupplungsende auch mit dem Gewinde des mindestens einen Kupplungselements verschraubt werden.

Die eingangs gestellte Aufgabe wird ferner bei einem Wirbelsäulenstabilisierungssystem der eingangs beschriebenen Art erfindungsgemäß dadurch gelöst, dass mindestens eine der mindestens zwei Knochenschrauben in Form eines der oben beschriebenen Pedikelschraubensysteme ausgebildet ist.

Ein derart weitergebildetes Wirbelsäulenstabilisierungssystem weist dann insbesondere auch die oben im Zusammenhang mit bevorzugten Ausführungsformen von Pedikelschraubensystemen beschriebenen Vorteile auf.

Die nachfolgende Beschreibung bevorzugter Ausführungsformen der Erfindung dient im Zusammenhang mit den Zeichnungen der näheren Erläuterung. Es zeigen:
- Figur 1:: eine schematische Ansicht eines Wirbelsäulenstabilisierungssystems umfassend zwei Knochenschrauben und ein Verbindungselement, welches an einer Wirbelsäule festgelegt ist;
- Figur 2:: eine Seitenansicht eines in einen Wirbel eingeschraubten Pedikelschraubensystems;
- Figur 3:: eine weitere Seitenansicht des in Figur 2 dargestellten Pedikelschraubensystems;
- Figur 4:: eine schematische perspektivische Ansicht des Pedikelschraubensystems aus Figur 2 umfassend eine Pedikelschraube mit daran gekoppelter Knochenausrichteinrichtung;
- Figur 5:: eine Seitenansicht des Pedikelschraubensystems aus Figur 4 mit geschnittener Knochenausrichteinrichtung und geschnittenem Schraubenkopf;
- Figur 6:: eine weitere Seitenansicht des Pedikelschraubensystems aus Figur 4 mit geschnittener Knochenausrichteinrichtung und geschnittenem Schraubenkopf;

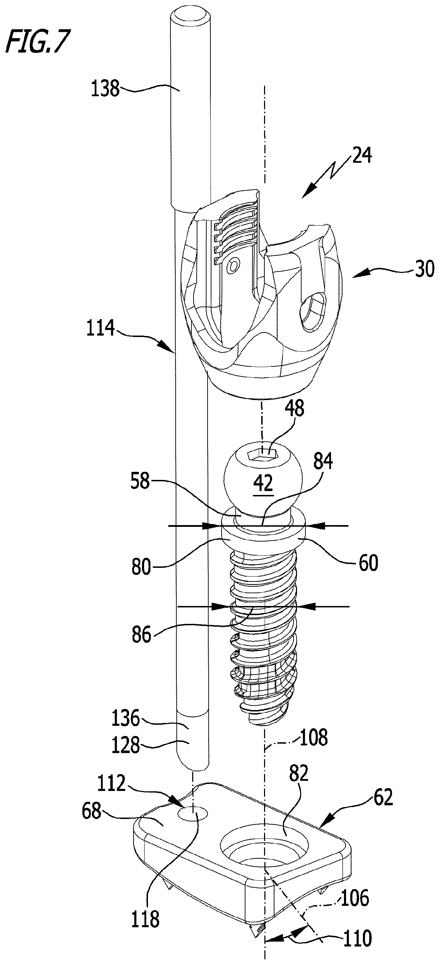

- Figur 7:: eine Explosionsdarstellung des Pedikelschraubensystems aus Figur 1 mit Ausrichtinstrument; und

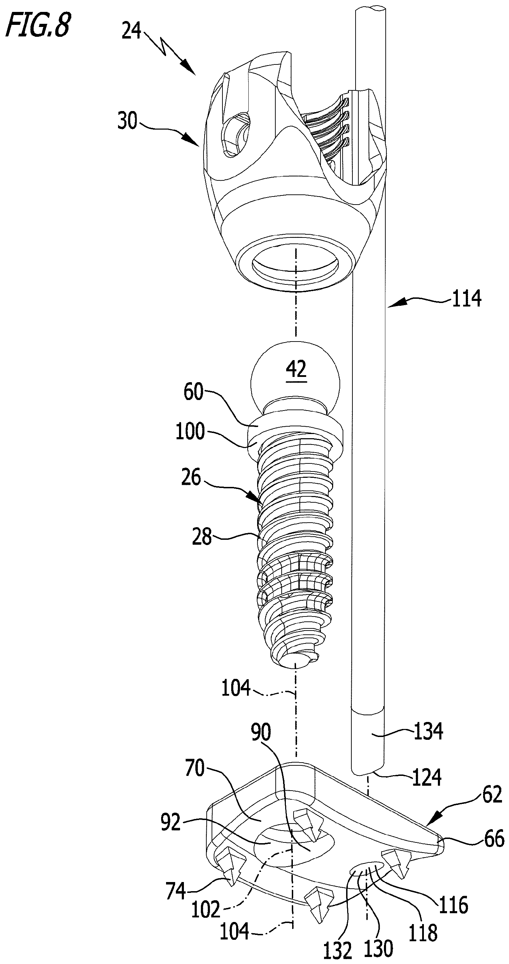

- Figur 8:: eine weitere perspektivische Explosionsdarstellung des Pedikelschraubensystems aus Figur 7.

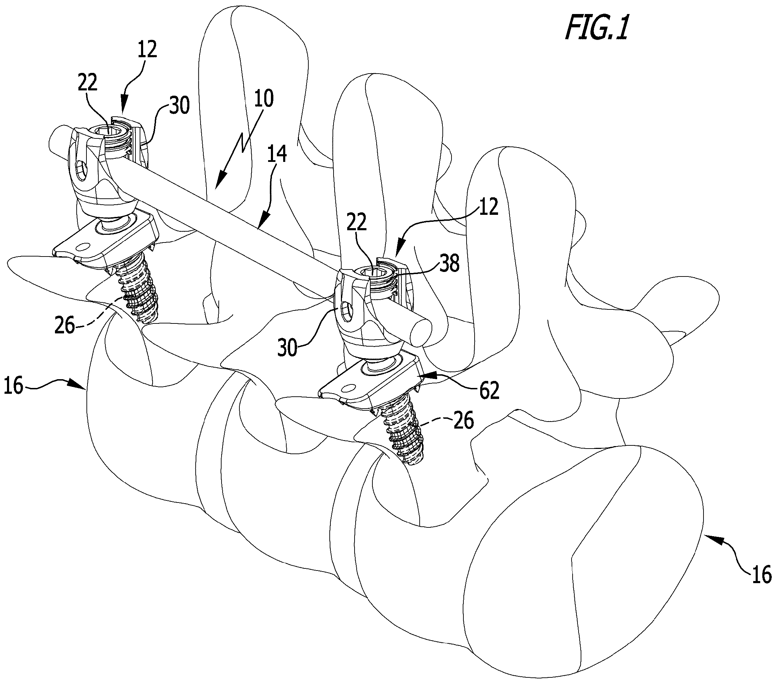

In Figur 1 ist beispielhaft ein insgesamt mit dem Bezugszeichen 10 bezeichnetes Wirbelsäulenstabilisierungssystem dargestellt, welches zwei Knochenschrauben 12 und ein an den beiden Knochenschrauben 12 festgelegtes Verbindungselement 14 umfasst. Die Knochenschrauben 12 sind an jeweils einem Wirbel 16 einer Wirbelsäule 18 festgelegt.

Selbstverständlich kann das Wirbelsäulenstabilisierungssystem 10 auch mehr als zwei Knochenschrauben 12 umfassen. Diese können beispielsweise über ein oder mehrere Verbindungselemente 14 miteinander verbunden werden.

In Figur 1 ist ein Verbindungselement 14 beispielhaft in Form eines Rundstabes dargestellt. Denkbar sind auch plattenförmige Verbindungselemente mit entsprechend ausgebildeten Abschnitten, die in Verbindungselementaufnahmen der Knochenschrauben eingesetzt und beispielsweise mit einer Fixierschraube 22 festgelegt werden können.

Bei den Knochenschrauben 12 kann es sich grundsätzlich um herkömmliche, am Markt verfügbare Pedikelschrauben handeln. Vorzugsweise ist mindestens eine der Knochenschrauben 12 jedoch in Form eines Pedikelschraubensystems 20 ausgebildet, welches nachfolgend im Einzelnen näher erläutert wird.

Jedes der Pedikelschraubensysteme 20 umfasst eine Pedikelschraube 24 mit einem Schraubenschaft 26, welcher ein Außengewinde 28 aufweist, beispielsweise in Form eines selbstschneidenden Knochengewindes, und einen kugelgelenkig am Schraubenschaft 26 gelagerten Schraubenkopf 30. Der Schraubenkopf 30 weist eine zwischen zwei freien Schenkeln 32 gebildete Verbindungselementaufnahme 34 für das Verbindungselement 14 des Wirbelsäulenstabilisierungssystems 10 auf.

An der Verbindungselementaufnahme 34 ist ferner ein Innengewinde 36 vorgesehen, welches korrespondierend zu einem Außengewinde 38 der Fixierschraube 22 ausgebildet ist, sodass die Fixierschraube zum Festlegen des Verbindungselements 14 ausgehend von freien Enden der Schenkel 32 in die Verbindungselementaufnahme 34 eingeschraubt werden kann, um das Verbindungselement 14 am Schraubenkopf 30 festzulegen.

Zur Ausbildung eines Kugelgelenks 40 zwischen dem Schraubenschaft 26 und dem Schraubenkopf 30 ist ein proximales Ende des Schraubenschafts 26 in Form eines Gelenkkopfs 42 ausgebildet mit einer in proximaler Richtung weisenden ebenen Endfläche 44, an welche eine einen Teil einer Kugeloberfläche bildende Gelenkkopffläche 46 angrenzt. In proximaler Richtung weisend ist im Gelenkkopf 42 eine Werkzeugelementaufnahme 48 ausgebildet, beispielsweise in Form eines Innenvielkant oder eines Innenvielrund.

Am Schraubenkopf 30 ist eine Gelenkkopfaufnahme 50 ausgebildet in Form eines korrespondierend zum Gelenkkopf 42 ausgebildeten Sitzes 52, welcher in eine in distaler Richtung sich im Innendurchmesser verjüngende Durchbrechung 54 mündet, die an einem distalen Ende 56 des Schraubenkopfs 30 ausgebildet ist und aus der der Schraubenschaft 26 distalseitig des Gelenkkopfs 42 vorsteht.

Distalseitig schließt sich an den Gelenkkopf 42 ein gewindefreier Schaftabschnitt 58 an, welcher distalseitig von einem Ringflansch 60 begrenzt ist. Der Ringflansch 60 weist einen Außendurchmesser auf, der etwas größer ist als ein Außendurchmesser des Gelenkkopfs 42.

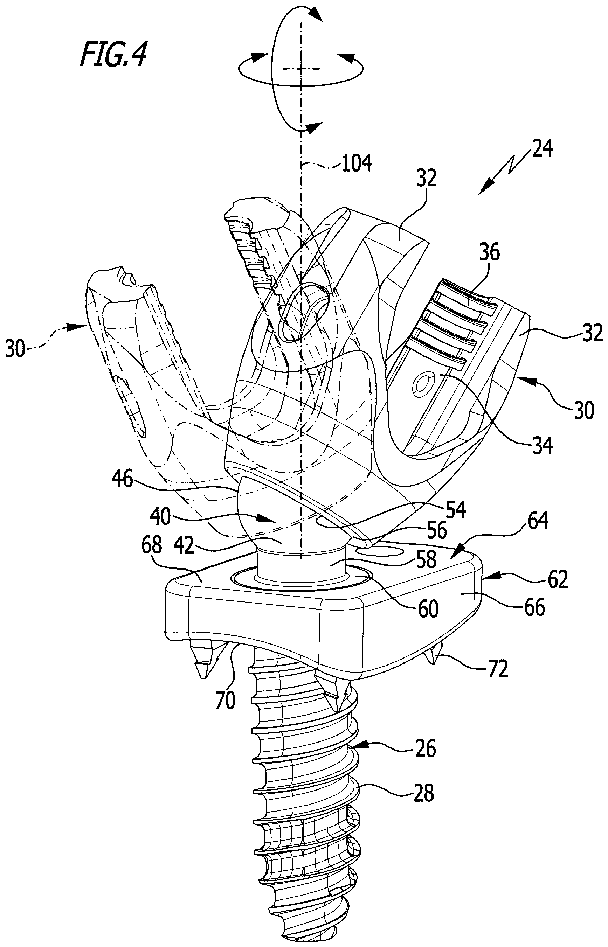

Das Pedikelschraubensystem 20 umfasst ferner eine Knochenausrichteinrichtung 62 und eine Kopplungseinrichtung 64 zum kraft- und/oder formschlüssigen Koppeln der Knochenausrichteinrichtung 62 und des Schraubenschafts 26 in einer Ausrichtstellung, wie sie beispielsweise in den Figuren 1 bis 6 beispielhaft dargestellt ist.

Die Knochenausrichteinrichtung 62 ist in Form einer Knochenplatte 66 ausgebildet, die eine ebene Oberseite 68 und konkavokonvex gekrümmte, vorzugsweise an eine Kontur eines Pedikels angepasste Unterseite 70 aufweist. Von der Unterseite der Knochenausrichteinrichtung 62 stehen mehrere Knochenverankerungselemente 72 ab, die in Form von dornenartigen Knochenpins 74 ausgebildet sind. Diese können optional auch widerhakenartige Verzahnungen aufweisen. Vorzugsweise sind die Knochenverankerungselemente 72 einstückig mit der Knochenplatte 66 ausgebildet.

Zum Koppeln des Schraubenschafts 26 mit der Knochenausrichteinrichtung 62 umfasst die Kopplungseinrichtung 64 erste und zweite Kopplungselemente 76 und 78, die in der Ausrichtstellung kraft- und/oder formschlüssig in Eingriff stehen und einerseits an der Knochenausrichteinrichtung 62 und andererseits am Schraubenschaft 26 angeordnet oder ausgebildet sind.

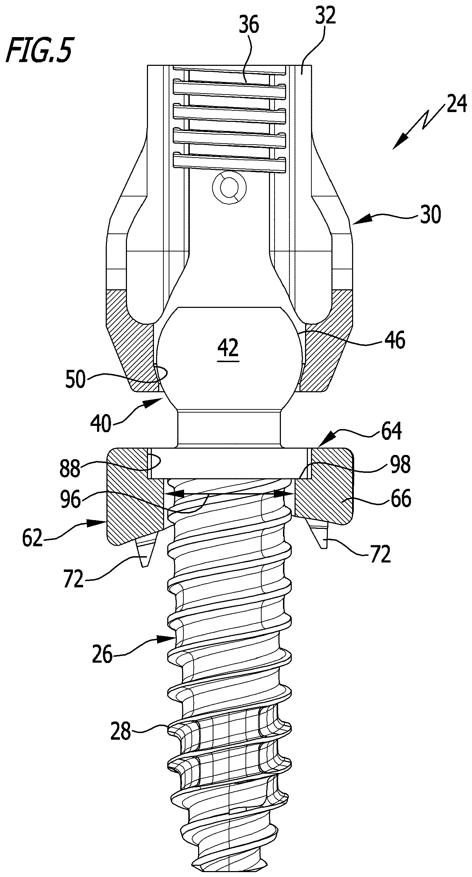

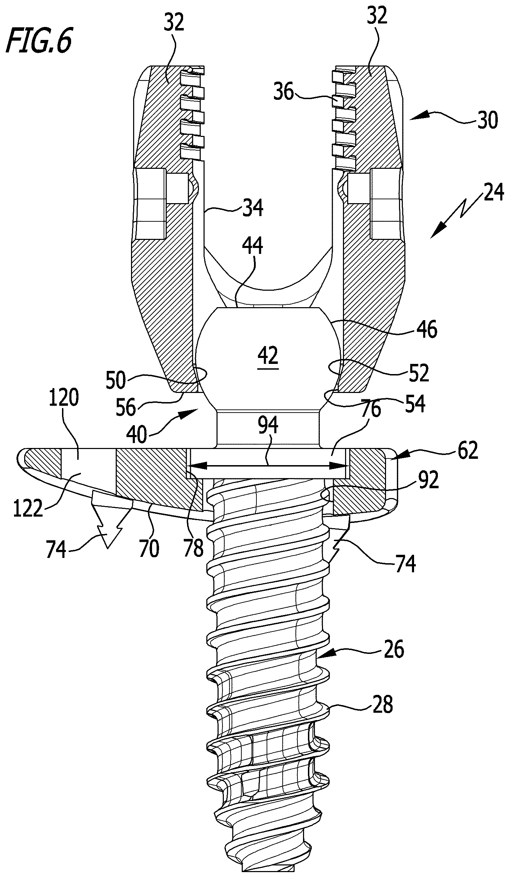

Das erste Kopplungselement 76 ist in Form eines Kopplungsvorsprungs 80 am Schraubenschaft 26 ausgebildet, das zweite Kopplungselement 78 in Form einer zum Kopplungsvorsprung 80 korrespondierenden Kopplungsaufnahme 82 an der Knochenausrichteinrichtung 62. Wie beispielsweise in den Figuren 5 und 6 gut zu erkennen, greift der Kopplungsvorsprung 80 in der Ausrichtstellung formschlüssig beziehungsweise im Wesentlichen formschlüssig in die Kopplungsaufnahme 82 ein.

Bei dem in den Figuren dargestellten Ausführungsbeispiel des Pedikelschraubensystems 20 ist der Kopplungsvorsprung 80 durch den Ringflansch 60 ausgebildet. Dieser kann insbesondere einstückig mit dem Schraubenschaft 26 ausgebildet sein. Alternativ kann der Kopplungsvorsprung 80 mit dem Schraubenschaft 26 auch kraft- und/oder stoffschlüssig verbunden sein, beispielsweise verpresst, verklebt, verlötet oder verschweißt.

Wie in den Figuren gut zu erkennen, ist der Kopplungsvorsprung 80 zwischen dem Außengewinde 28 und dem Gelenkkopf 42 angeordnet. Zudem ist ein Außendurchmesser 84 des Kopplungsvorsprungs 80 auch größer als ein maximaler Außendurchmesser 86 des Außengewindes 28.

Die Kopplungsaufnahme 82 ist in Form einer Ausnehmung 88 der Knochenausrichteinrichtung 62 ausgebildet.

Die Knochenausrichteinrichtung 62 weist ferner eine Schraubenschaftaufnahme 90 auf, die der Schraubenschaft in der Ausrichtstellung durchsetzt. Sie ist in Form einer Durchbrechung 92 der Knochenplatte 66 ausgebildet. Die Kopplungsaufnahme 82 grenzt direkt an die Schraubenschaftaufnahme 90 an.

Ein Innendurchmesser 94 der Kopplungsaufnahme 82 ist größer als ein Innendurchmesser der Schraubenschaftaufnahme 90. Durch diese Wahl der Abmessungen der Kopplungsaufnahme 82 und der Schraubenschaftaufnahme 90 wird eine sich einstufig in distaler Richtung verjüngende Durchbrechung ausgebildet, die eine in proximaler Richtung weisende, die Kopplungsaufnahme 82 teilweise begrenzende Ringfläche 98 definiert, an welcher eine in distaler Richtung weisende Ringfläche 100 des Ringflanschs 60 in der Ausrichtstellung anliegt beziehungsweise anschlägt.

Bei dem in den Figuren dargestellten Ausführungsbeispiel definiert die Kopplungseinrichtung 64 eine Längsachse 102, welche parallel zu einer Schraubenschaftlängsachse 104 des Schraubenschafts 26 verläuft beziehungsweise mit dieser zusammenfällt.

Alternativ ist es auch denkbar, eine Längsachse der Kopplungseinrichtung 64, die beispielsweise vorgegeben ist durch eine Längsachse 106 der Schraubenschaftaufnahme 90, relativ zu einer Längsachse 108, die durch die Kopplungsaufnahme 82 vorgegeben ist, um einen Neigungswinkel 110 geneigt auszubilden. Der Neigungswinkel 110 kann beispielsweise einen Wert in einem Bereich von etwa 0° bis etwa 30° aufweisen.

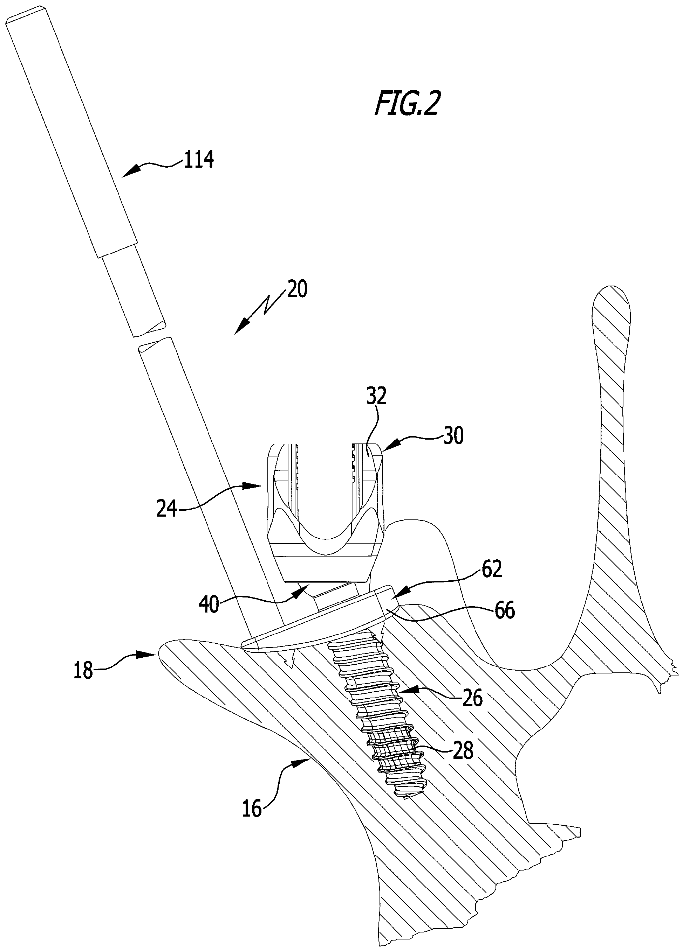

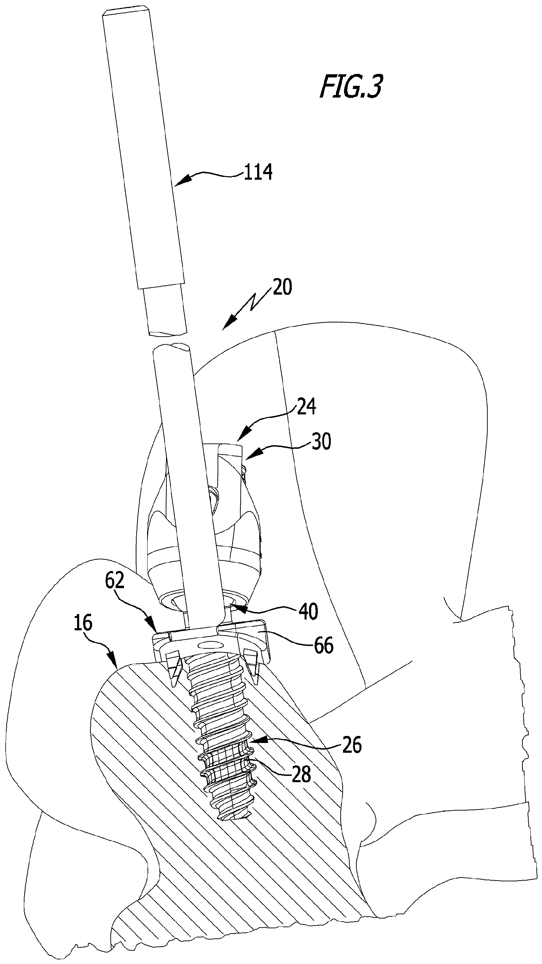

Ferner umfasst die Knochenausrichteinrichtung 62 vorzugsweise mindestens eine Kupplungseinrichtung 112 zum temporären kraft- und/oder formschlüssigen Kuppeln mit einem Ausrichtinstrument 114 in einer beispielsweise in den Figuren 2 und 3 dargestellten Kuppelstellung.

Die Kupplungseinrichtung 112 umfasst mindestens ein Kupplungselement 116 zum kraft- und/oder formschlüssigen Kuppeln mit dem Ausrichtinstrument 114 in der Kuppelstellung. Das Kupplungselement 116 kann insbesondere in Form eines Kupplungsvorsprungs oder, wie in den Figuren beispielhaft dargestellt, in Form einer Kupplungsausnehmung 118 ausgebildet sein. Ferner ist es alternativ auch möglich, sowohl einen Kupplungsvorsprung als auch eine Kupplungsausnehmung an der Knochenausrichteinrichtung 62 vorzusehen.

Die Kupplungsausnehmung 118 ist bei dem in den Figuren dargestellten Ausführungsbeispiel in Form einer Durchbrechung 120 der Knochenplatte 66 ausgebildet. Alternativ ist es möglich, die Kupplungsausnehmung 118 auch in Form eines Sacklochs auszubilden. Vorzugsweise wird die Durchbrechung 120 in Form einer Bohrung 122 ausgebildet.

Um eine einfache und sichere Verbindung zwischen dem Ausrichtinstrument 114 und der Knochenausrichteinrichtung 62 zu realisieren, ist ausgehend von einem distalen Ende 124 des Ausrichtinstruments 114 ein Kupplungsende 128 zum kraft- und/oder formschlüssigen Kuppeln mit der Kupplungseinrichtung 112 der Knochenausrichteinrichtung 62 ausgebildet. Hierfür kann das Kupplungselement 116, welches in Form der Bohrung 122 ausgebildet ist, ferner mit einem Gewinde 130, insbesondere einem Innengewinde 132, versehen oder in Form eines Rastelements ausgebildet sein. Das Kupplungsende 128 ist dann vorzugsweise korrespondierend zur Kupplungseinrichtung 112 ausgebildet, also beispielsweise ebenfalls in Form eines Rastelements oder ein Gewinde 134 in Form eines Außengewindes 136 umfassend.

Die Funktionsweise des Wirbelsäulenstabilisierungssystems 10 sowie dessen Pedikelschraubensysteme 20 wird nachfolgend erläutert.

Zum Einbringen der Pedikelschraube 24 in den Wirbel 16 wird mit einem in den Figuren nicht dargestellten Einschraubinstrument, welches ein zur Werkzeugelementaufnahme 48 korrespondierendes Werkzeugende aufweist, der Schraubenschaft 26 in den Wirbel 16 eingeschraubt. Hierfür kann der Wirbel vorab angebohrt oder anderweitig mit einer Führungsöffnung versehen werden.

Vor dem Einschrauben kann die Knochenausrichteinrichtung 62 bereits mit dem Schraubenschaft 26 gekoppelt werden, sodass der Schraubenschaft 26 und die Knochenausrichteinrichtung 62 bereits die Ausrichtstellung einnehmen. Denkbar ist es alternativ, die Knochenplatte 66 an einer gewünschten Stelle des Wirbels 16 anzubringen, beispielsweise durch Einschlagen der Knochenverankerungselemente 72 in den Wirbel 16. Der Schraubenschaft 26 kann dann durch die Kopplungsaufnahme 82 und die Schraubenschaftaufnahme 90 hindurch in den Wirbel 16 eingeschraubt werden, bis der Ringflansch 60 an der Ringfläche 98 der Kopplungsaufnahme 82 anschlägt.

Um eine sichere Orientierung und Befestigung der Pedikelschraube 24 am Wirbel 16 zu erreichen, kann optional bereits beim Einschrauben der Pedikelschraube 24 das Ausrichtinstrument 114 mit der Knochenplatte 66 in der oben beschriebenen Weise verbunden werden, indem das Kupplungsende 128 und die Kupplungseinrichtung 112 kraft- und/oder formschlüssig miteinander in Eingriff gebracht werden. Ein Operateur kann so ein proximales Ende des Ausrichtinstruments 114, welches vorzugsweise in Form eines Griffelements 138 ausgebildet ist, erfassen und damit die Knochenplatte 66 in einer gewünschten Orientierung ausrichten und in dieser halten.

Ist die Pedikelschraube 24, wie beispielhaft in den Figuren 2 und 3 schematisch dargestellt, im Wirbel 16 verankert, kann ein Operateur durch Manipulieren des Ausrichtinstruments 114 den Wirbel 16 insgesamt bewegen und in eine gewünschte Stellung bringen, um die Wirbelsäule 18 wieder in eine vorgesehene Form zu bringen.

In der beschriebenen Weise kann jede der Pedikelschrauben 24 mit einem Ausrichtinstrument 114 gekoppelt und gleichzeitig bewegt werden.

Dadurch, dass die Schraubenköpfe 30 und die Schraubenschäfte 26 über das Kugelgelenk 40 miteinander kugelgelenkig gekoppelt sind, kann das Verbindungselement 14 anschließend auf einfache Weise in die Verbindungselementaufnahmen 34 eingesetzt und mit den Fixierschrauben festgelegt werden. Die Schraubenköpfe 30 können nämlich um einen Mittelpunkt des Gelenkkopfs 42 relativ zu den Schraubenschäften 26 verschwenkt und so ausgerichtet werden, dass beispielsweise ein stabförmiges Verbindungselement 14 ohne weitere Verformung der Pedikelschrauben 24 in die Verbindungselementaufnahmen 34 eingelegt werden kann.

Das vorgeschlagene Pedikelschraubensystem 20 vereint somit die Vorteile einer Polyaxialschraube einerseits und andererseits eine mögliche Manipulationsmöglichkeit von Wirbeln, in die die Pedikelschraube eingeschraubt ist, wie sie insbesondere bei Pedikelschrauben mit unbeweglich am Schraubenschaft angeordneten Schraubenköpfen oder bei Monoaxialschrauben der Fall ist, miteinander.

### Bezugszeichenliste

- 10: Wirbelsäulenstabilisierungssystem
- 12: Knochenschraube
- 14: Verbindungselement
- 16: Wirbel
- 18: Wirbelsäule
- 20: Pedikelschraubensystem
- 22: Fixierschraube
- 24: Pedikelschraube
- 26: Schraubenschaft
- 28: Außengewinde
- 30: Schraubenkopf
- 32: Schenkel
- 34: Verbindungselementaufnahme
- 36: Innengewinde
- 38: Außengewinde
- 40: Kugelgelenk
- 42: Gelenkkopf
- 44: Endfläche
- 46: Gelenkkopffläche
- 48: Werkzeugelementaufnahme
- 50: Gelenkkopfaufnahme
- 52: Sitz
- 54: Durchbrechung
- 56: Ende
- 58: Schaftabschnitt
- 60: Ringflansch
- 62: Knochenausrichteinrichtung
- 64: Kopplungseinrichtung
- 66: Knochenplatte
- 68: Oberseite
- 70: Unterseite
- 72: Knochenverankerungselement
- 74: Knochenpin
- 76: erstes Kopplungselement
- 78: zweites Kopplungselement
- 80: Kopplungsvorsprung
- 82: Kopplungsaufnahme
- 84: Außendurchmesser
- 86: Außendurchmesser
- 88: Ausnehmung
- 90: Schraubenschaftaufnahme
- 92: Durchbrechung
- 94: Innendurchmesser
- 96: Innendurchmesser
- 98: Ringfläche
- 100: Ringfläche
- 102: Längsachse
- 104: Schraubenschaftlängsachse
- 106: Längsachse
- 108: Längsachse
- 110: Neigungswinkel
- 112: Kupplungseinrichtung
- 114: Ausrichtinstrument
- 116: Kupplungselement
- 118: Kupplungsausnehmung
- 120: Durchbrechung
- 122: Bohrung
- 124: Ende
- 128: Kupplungsende
- 130: Gewinde
- 132: Innengewinde
- 134: Gewinde
- 136: Außengewinde
- 138: Griffelement

## Patentansprüche

1. Pedikelschraubensystem (20) umfassend eine Pedikelschraube (24) mit einem Schraubenschaft (26), welcher ein Außengewinde (28) aufweist, und einem kugelgelenkig am Schraubenschaft (26) gelagerten Schraubenkopf (30), welcher Schraubenkopf (30) eine Verbindungselementaufnahme (34) für ein Verbindungselement (14) eines Wirbelsäulenstabilisierungssystems (10) umfasst, das Pedikelschraubensystem (20) ferner umfassend eine Knochenausrichteinrichtung (62) und eine Kopplungseinrichtung (64) zum kraft- und/oder formschlüssigen Koppeln der Knochenausrichteinrichtung (62) und des Schraubenschafts (26) in einer Ausrichtstellung,
wobei die Knochenausrichteinrichtung (62) in Form einer Knochenplatte (66) ausgebildet ist,
wobei die Kopplungseinrichtung (64) erste und zweite Kopplungselemente (76, 78) umfasst, welche in der Ausrichtstellung kraft- und/oder formschlüssig in Eingriff stehen und einerseits an der Knochenausrichteinrichtung (62) und andererseits am Schraubenschaft (26) angeordnet oder ausgebildet sind,
wobei das erste Kopplungselement (76) in Form eines Kopplungsvorsprungs (80) und wobei das zweite Kopplungselement (78) in Form einer zum Kopplungsvorsprung (80) korrespondierenden Kopplungsaufnahme (82) ausgebildet ist,
**dadurch gekennzeichnet, dass** der Kopplungsvorsprung (80) in der Ausrichtstellung formschlüssig oder im Wesentlichen formschlüssig in die Kopplungsaufnahme (82) eingreift,
dass der Kopplungsvorsprung (80) in Form eines Ringflansches (60) ausgebildet ist,
dass die Kopplungsaufnahme (82) in Form einer Ausnehmung (88) der Knochenausrichteinrichtung (62) ausgebildet ist,
dass die Knochenausrichteinrichtung (62) mindestens eine Kupplungseinrichtung (112) zum temporären kraft- und/oder formschlüssigen Kuppeln mit einem Ausrichtinstrument (114) in einer Kuppelstellung aufweist, dass die Kupplungseinrichtung (112) mindestens ein Kupplungselement (116) zum kraft- und/oder formschlüssigen Kuppeln mit dem Ausrichtinstrument (114) in einer Kuppelstellung umfasst,
dass das mindestens eine Kupplungselement (116) in Form eines Kupplungsvorsprungs und/oder in Form einer Kupplungsausnehmung (118) ausgebildet ist,
dass das mindestens eine Kupplungselement (116) ein Gewinde (130) umfasst, insbesondere ein Außen- oder ein Innengewinde (132), oder in Form eines Rastelements ausgebildet ist,
dass das Pedikelschraubensystem ein Ausrichtinstrument (114) zum temporären kraft- und/oder formschlüssigen Kuppeln mit der Kupplungseinrichtung (112) umfasst und
dass das Ausrichtinstrument (114) distalseitig ein Kupplungsende (128) aufweist zum kraft- und/oder formschlüssigen Kuppeln mit der Kupplungseinrichtung (112).

2. Pedikelschraubensystem nach Anspruch 1, **dadurch gekennzeichnet, dass** die Knochenausrichteinrichtung (62) mindestens ein Knochenverankerungselement (72) trägt oder umfasst.

3. Pedikelschraubensystem nach Anspruch 2, **dadurch gekennzeichnet, dass** das mindestens eine Knochenverankerungselement (72) in Form eines Knochenpins (74) oder eines Knochenzahns ausgebildet ist und/oder
dass das mindestens eine Knochenverankerungselement (72) einstückig mit der Knochenausrichteinrichtung (62) ausgebildet ist.

4. Pedikelschraubensystem nach einem der voranstehenden Ansprüche, **dadurch gekennzeichnet, dass** der Kopplungsvorsprung (80) einstückig mit dem Schraubenschaft (26) ausgebildet oder kraft- und/oder stoffschlüssig mit dem Schraubenschaft (26) verbunden ist, insbesondere verpresst, verklebt, verlötet oder verschweißt.

5. Pedikelschraubensystem nach einem der voranstehenden Ansprüche, **dadurch gekennzeichnet, dass** der Schraubenschaft (26) einen Gelenkkopf (42) aufweist und dass der Schraubenkopf (30) eine zum Gelenkkopf (42) korrespondierende Gelenkkopfaufnahme (50) zur Ausbildung eines Kugelgelenks (40) im Zusammenwirken mit dem Gelenkkopf (42) aufweist.

6. Pedikelschraubensystem nach Anspruch 5, **dadurch gekennzeichnet, dass** der Kopplungsvorsprung (80) zwischen dem Außengewinde (28) und dem Gelenkkopf (42) angeordnet ist.

7. Pedikelschraubensystem nach einem der voranstehenden Ansprüche, **dadurch gekennzeichnet, dass** ein Außendurchmesser (84) des Kopplungsvorsprungs (80) größer ist als ein maximaler Außendurchmesser (86) des Außengewindes (28),
und/oder
dass der Kopplungsvorsprung (80) direkt an das Außengewinde (28) angrenzt.

8. Pedikelschraubensystem nach einem der voranstehenden Ansprüche, **dadurch gekennzeichnet, dass** die Knochenausrichteinrichtung (62) eine Schraubenschaftaufnahme (90) aufweist, in die der Schraubenschaft (26) in der Ausrichtstellung mindestens teilweise eingreift.

9. Pedikelschraubensystem nach Anspruch 8, **dadurch gekennzeichnet, dass** die Schraubenschaftaufnahme (90) in Form einer Durchbrechung (92) der Knochenausrichteinrichtung (62) ausgebildet ist.

10. Pedikelschraubensystem nach Anspruch 8 oder 9, **dadurch gekennzeichnet, dass** die Kopplungsaufnahme (82) direkt an die Schraubenschaftaufnahme (90) angrenzt.

11. Pedikelschraubensystem nach einem der Ansprüche 8 bis 10, **dadurch gekennzeichnet, dass** die Kopplungsaufnahme (82) einen größeren Innendurchmesser (94) aufweist als die Schraubenschaftaufnahme (90).

12. Pedikelschraubensystem nach einem der voranstehenden Ansprüche, **dadurch gekennzeichnet, dass** die Kopplungseinrichtung (64) eine Längsachse (102) definiert, welche parallel zu einer Schraubenschaftlängsachse (104) des Schraubenschafts (26) verläuft oder mit dieser einen Neigungswinkel (110) einschließt.

13. Pedikelschraubensystem nach einem der voranstehenden Ansprüche, **dadurch gekennzeichnet, dass** die Kupplungsausnehmung (118) in Form eines Sacklochs oder einer Durchbrechung (120) ausgebildet ist, insbesondere in Form einer Bohrung (122).

14. Pedikelschraubensystem nach einem der voranstehenden Ansprüche, **dadurch gekennzeichnet, dass** das Kupplungsende (128) in Form eines Rastelements ausgebildet ist oder ein Gewinde (134) umfasst, welches korrespondierend zum Gewinde (130) des mindestens einen Kupplungselements (116) ausgebildet ist.

15. Wirbelsäulenstabilisierungssystem (10) umfassend mindestens zwei Knochenschrauben (12) und mindestens ein an den mindestens zwei Knochenschrauben (12) festlegbares Verbindungselement (14), **dadurch gekennzeichnet, dass** mindestens eine der mindestens zwei Knochenschrauben (12) in Form eines Pedikelschraubensystems nach einem der voranstehenden Ansprüche ausgebildet ist.

## Claims

1. Pedicle screw system (20), comprising a pedicle screw (24) having a screw shaft (26) with an external thread (28) and having a screw head (30) supported on the screw shaft (26) in a ball-and-socket joint relationship therewith, which screw head (30) comprises a connecting element receptacle (34) for a connecting element (14) of a spinal stabilization system (10), the pedicle screw system (20) further comprising a bone alignment device (62) and a coupling device (64) for force-locking coupling and/or form-locking coupling of the bone alignment device (62) and the screw shaft (26) when in an alignment position,
wherein the bone alignment device (62) is configured in the form of a bone plate (66),
wherein the coupling device (64) comprises first and second coupling elements (76, 78) which are in force-locking engagement and/or form-locking engagement when in the alignment position and are arranged or formed on the bone alignment device (62) on the one hand and the screw shaft (26) on the other,
wherein the first coupling element (76) is configured in the form of a coupling projection (80) and wherein the second coupling element (78) is configured in the form of a coupling receptacle (82) corresponding to the coupling projection (80),
**characterized in that** the coupling projection (80) engages in the coupling receptacle (82) in a form-locking manner or in an essentially form-locking manner when in the alignment position,
**in that** the coupling projection (80) is configured in the form of an annular flange (60),
**in that** the coupling receptacle (82) is configured in the form of a recess (88) of the bone alignment device (62),
**in that** the bone alignment device (62) has at least one coupling device (112) for temporary force-locking coupling and/or temporary form-locking coupling with an alignment instrument (114) in a coupling position,
**in that** the coupling device (112) has at least one coupling element (116) for force-locking coupling and/or form-locking coupling with the alignment instrument (114) in a coupling position,
**in that** the at least one coupling element (116) is configured in the form of a coupling projection and/or in the form of a coupling recess (118), **in that** the at least one coupling element (116) comprises a thread (130), particularly an external thread or an internal thread (132), or is configured in the form of a latch element,
**in that** the pedicle screw system comprises an alignment instrument (114) for temporary force-locking coupling and/or temporary form-locking coupling with the coupling device (112) and
**in that** the alignment instrument (114) comprises on the distal side thereof a coupling end (128) for force-locking coupling and/or form-locking coupling with the coupling device (112).

2. Pedicle screw system in accordance with claim 1, **characterized in that** the bone alignment device (62) carries or comprises at least one bone anchoring element (72).

3. Pedicle screw system in accordance with claim 2, **characterized in that** the at least one bone anchoring element (72) is configured in the form of a bone pin (74) or a bone tooth
and/or
**in that** the at least one bone anchoring element (72) is configured in one piece with the bone alignment device (62).

4. Pedicle screw system in accordance with any one of the preceding claims, **characterized in that** the coupling projection (80) is configured in one piece with the screw shaft (26) or is connected to the screw shaft (26) in a force-locking manner and/or with a substance-to-substance bond, in particular by press-fitting, adhesive bonding, soldering or welding.

5. Pedicle screw system in accordance with any with any one of the preceding claims, **characterized in that** the screw shaft (26) has a joint head (42) and **in that** the screw head (30) has a joint head receptacle (50) corresponding to the joint head (42) for forming a ball-and-socket joint (40) in cooperation with the joint head (42).

6. Pedicle screw system in accordance with claim 5, **characterized in that** the coupling projection (80) is arranged between the external thread (28) and the joint head (42).

7. Pedicle screw system in accordance with any one of the preceding claims, **characterized in that** an external diameter (84) of the coupling projection (80) is larger than a maximum external diameter (86) of the external thread (28),
and/or
**in that** the coupling projection (80) is directly adjacent to the external thread (28).

8. Pedicle screw system in accordance with any one of the preceding claims, **characterized in that** the bone alignment device (62) comprises a screw shaft receptacle (90) in which the screw shaft (26) engages at least partially when in the alignment position.

9. Pedicle screw system in accordance with claim 8, **characterized in that** the screw shaft receptacle (90) is configured in the form of a through-hole (92) of the bone alignment device (62).

10. Pedicle screw system in accordance with claim 8 or 9, **characterized in that** the coupling receptacle (82) is directly adjacent to the screw shaft receptacle (90).

11. Pedicle screw system in accordance with any one of claims 8 to 10, **characterized in that** an internal diameter (94) of the coupling receptacle (82) is larger than an internal diameter of the screw shaft receptacle (90).

12. Pedicle screw system in accordance with any one of the preceding claims, **characterized in that** the coupling device (64) defines a longitudinal axis (102) which runs parallel to a screw shaft longitudinal axis (104) of the screw shaft (26) or encloses an angle of inclination (110) therewith.

13. Pedicle screw system in accordance with any one of the preceding claims, **characterized in that** the coupling recess (118) is configured in the form of a blind hole or a through-hole (120), particularly in the form of a bore (122).

14. Pedicle screw system in accordance with any one of the preceding claims, **characterized in that** the coupling end (128) is configured in the form of a latch element or comprises a thread (134) which is of a configuration corresponding to that of the thread (130) of the at least one coupling element (116).

15. Spinal stabilization system (10), comprising at least two bone screws (12) and at least one connecting element (14) fixable on the at least two bone screws (12), **characterized in that** at least one of the at least two bone screws (12) is configured in the form of a pedicle screw system in accordance with any one of the preceding claims.

## Revendications

1. Système de vis pédiculaire (20) comprenant une vis pédiculaire (24) avec un corps de vis (26), qui présente un filetage extérieur (28), et une tête de vis (30) montée par une articulation à rotule sphérique sur le corps de vis (26), ladite tête de vis (30) comportant un logement d'accueil d'élément de liaison (34) pour un élément de liaison (14) d'un système de stabilisation de colonne vertébrale (10), le système de vis pédiculaire (20) comprenant, en outre, un dispositif d'orientation d'os (62) et un dispositif de couplage (64) pour le couplage par adhérence et/ou complémentarité de formes du dispositif d'orientation d'os (62) et du corps de vis (26), dans une position d'orientation,
système de vis pédiculaire
dans lequel le dispositif d'orientation d'os (62) est réalisé sous la forme d'une plaque à os (66),
dans lequel le dispositif de couplage (64) comporte des premiers et deuxièmes éléments de couplage (76, 78), qui, dans la position d'orientation, sont en prise réciproque par adhérence et/ou complémentarité de formes, et sont agencés ou formés d'une part sur le dispositif d'orientation d'os (62) et d'autre part sur le corps de vis (26),
dans lequel le premier élément de couplage (76) est réalisé sous la forme d'une proéminence de couplage (80), et le deuxième élément de couplage (78) sous la forme d'un logement de couplage (82) correspondant à la proéminence de couplage (80),
**caractérisé en ce que** dans la position d'orientation, la proéminence de couplage (80) s'engage par complémentarité de formes ou sensiblement par complémentarité de formes, dans le logement de couplage (82),
**en ce que** la proéminence de couplage (80) est réalisée sous la forme d'un flasque annulaire (60),
**en ce que** le logement de couplage (82) est réalisé sous la forme d'un évidement (88) du dispositif d'orientation d'os (62),
**en ce que** le dispositif d'orientation d'os (62) comporte au moins un dispositif d'accouplement (112) pour l'accouplement temporaire par adhérence et/ou complémentarité de formes avec un instrument d'orientation (114), dans une position d'accouplement, **en ce que** le dispositif d'accouplement (112) comporte au moins un élément d'accouplement (116) pour l'accouplement par adhérence et/ou complémentarité de formes avec l'instrument d'orientation (114), dans une position d'accouplement,
**en ce que** ledit au moins un élément d'accouplement (116) est réalisé sous la forme d'une proéminence d'accouplement et/ou sous la forme d'un logement d'accouplement (118), **en ce que** ledit au moins un élément d'accouplement (116) comporte un filetage (130), notamment un filetage extérieur ou intérieur (132), ou bien est réalisé sous la forme d'un élément d'encliquetage,
**en ce que** le système de vis pédiculaire comprend un instrument d'orientation (114) destiné à être accouplé temporairement par adhérence et/ou complémentarité de formes au dispositif d'accouplement (112), et
**en ce que** l'instrument d'orientation (114) présente, du côté distal, une extrémité d'accouplement (128) pour l'accouplement par adhérence et/ou complémentarité de formes avec le dispositif d'accouplement (112).

2. Système de vis pédiculaire selon la revendication 1, **caractérisé en ce que** le dispositif d'orientation d'os (62) porte ou comprend au moins un élément d'ancrage à l'os (72).

3. Système de vis pédiculaire selon la revendication 2, **caractérisé en ce que** ledit au moins un élément d'ancrage à l'os (72) est réalisé sous la forme d'un picot à os (74) ou d'une dent à os,
et/ou
**en ce que** ledit au moins un élément d'ancrage à l'os (72) est réalisé d'un seul tenant avec le dispositif d'orientation d'os (62).

4. Système de vis pédiculaire selon l'une des revendications précédentes, **caractérisé en ce que** la proéminence de couplage (80) est réalisée d'un seul tenant avec le corps de vis (26), ou est reliée par adhérence et/ou continuité de matière au corps de vis (26), notamment par montage à force, par collage, brasage ou soudage.

5. Système de vis pédiculaire selon l'une des revendications précédentes, **caractérisé en ce que** le corps de vis (26) présente une tête d'articulation (42), et **en ce que** la tête de vis (30) présente un logement d'accueil de tête d'articulation (50) correspondant à la tête d'articulation (42) pour former une articulation à rotule sphérique (40) par interaction avec la tête d'articulation (42) .

6. Système de vis pédiculaire selon la revendication 5, **caractérisé en ce que** la proéminence de couplage (80) est agencée entre le filetage extérieur (28) et la tête d'articulation (42).

7. Système de vis pédiculaire selon l'une des revendications précédentes, **caractérisé en ce qu'**un diamètre extérieur (84) de la proéminence de couplage (80) est supérieur à un diamètre extérieur maximal (86) du filetage extérieur (28),
et/ou
**en ce que** la proéminence de couplage (80) est directement adjacente au filetage extérieur (28).

8. Système de vis pédiculaire selon l'une des revendications précédentes, **caractérisé en ce que** le dispositif d'orientation d'os (62) comporte un logement d'accueil de corps de vis (90) dans lequel vient s'engager, au moins partiellement, le corps de vis (26), dans la position d'orientation.

9. Système de vis pédiculaire selon la revendication 8, **caractérisé en ce que** le logement d'accueil de corps de vis (90) est réalisé sous la forme d'une ouverture de passage (92) du dispositif d'orientation d'os (62).

10. Système de vis pédiculaire selon la revendication 8 ou la revendication 9, **caractérisé en ce que** le logement de couplage (82) est directement adjacent au logement d'accueil de corps de vis (90).

11. Système de vis pédiculaire selon l'une des revendications 8 à 10, **caractérisé en ce que** le logement de couplage (82) présente un diamètre intérieur (94) plus grand que le logement d'accueil de corps de vis (90).

12. Système de vis pédiculaire selon l'une des revendications précédentes, **caractérisé en ce que** le dispositif de couplage (64) définit un axe longitudinal (102), qui s'étend parallèlement à un axe longitudinal de corps de vis (104) du corps de vis (26), ou forme un angle d'inclinaison (110) avec celui-ci.

13. Système de vis pédiculaire selon l'une des revendications précédentes, **caractérisé en ce que** le logement d'accouplement (118) est réalisé sous la forme d'un trou borgne ou d'une ouverture de passage (120), notamment sous la forme d'un alésage (122).

14. Système de vis pédiculaire selon l'une des revendications précédentes, **caractérisé en ce que** l'extrémité d'accouplement (128) est réalisée sous la forme d'un élément d'encliquetage ou comprend un filetage (134), qui est réalisé de manière à correspondre au filetage (130) dudit au moins un élément d'accouplement (116) .

15. Système de stabilisation de colonne vertébrale (10) comprenant au moins deux vis à os (12) et au moins un élément de liaison (14) pouvant être fixé aux dites au moins deux vis à os (12), **caractérisé en ce que** l'une au moins des deux vis à os (12) est réalisée sous la forme d'un système de vis pédiculaire selon l'une des revendications précédentes.
